(19) Europäisches Patentamt
European Patent Office
Office européen des brevets

(11) Publication number: **0 097 592**
**B1**

(12) # EUROPEAN PATENT SPECIFICATION

(45) Date of publication of patent specification: **10.12.86**

(51) Int. Cl.⁴: **C 07 C 85/11**

(21) Application number: **83401267.6**

(22) Date of filing: **17.06.83**

(54) **Process for catalytically reducing nitroaromatic compounds.**

(30) Priority: **21.06.82 IT 2195382**

(43) Date of publication of application:
**04.01.84 Bulletin 84/01**

(45) Publication of the grant of the patent:
**10.12.86 Bulletin 86/50**

(84) Designated Contracting States:
**BE DE FR GB NL**

(56) References cited:
**FR-A-2 450 798**
**US-A-3 944 615**

**CHEMISTRY LETTERS, 1981, The Chemical Society of Japan, pages 1083-1986 T. OKANO et al.: "Application of the water-gas shift reaction. Reduction of nitrobenzenes with CO and H2O catalyzed by Ru(II) complexes"**

**CHEMICAL ABSTRACTS, vol. 95, no. 22, 30th November 1981, page 391, no. 192961v, Columbus, Ohio, USA K. KANEDA et al.: "Facile reduction of nitrobenzene using carbon monoxide and water catalyzed by rhodium carbonyl cluster-amine systems"**

(73) Proprietor: **Montedison S.p.A.**
**31, Foro Buonaparte**
**I-20121 Milan (IT)**

(72) Inventor: **Mestroni, Giovanni**
**27 Vicolo Castagneto**
**Trieste (IT)**
Inventor: **Zassinovich, Grazia**
**27 Vicolo Castagneto**
**Trieste (IT)**
Inventor: **Alessio, Enzo**
**12 Via Alfieri**
**Gorizia (IT)**

(74) Representative: **Hirsch, Marc-Roger**
**Cabinet Hirsch 34 rue de Bassano**
**F-75008 Paris (FR)**

(56) References cited:
**CHEMICAL ABSTRACTS, vol. 91, no. 7, 13th August 1979, page 616, no. 55934s, Columbus, Ohio, USA R.C. RYAN et al.: "Metal cluster catalysis. 2. Selective reduction of nitrobenzene catalyzed by rhodium carbonyl cluster anions. Evidence for water gas shift reaction"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 097 592 B1

(58) References cited:
**CHEMICAL ABSTRACTS, vol. 98, no. 17, 25th April 1983, page 523, no. 142762x, Columbus, Ohio, USA E. ALESSIO et al.: "Catalytic reduction of nitroaromatic compounds with carbon monoxide and water using 3,4,7,8-Me4phen-carbonyl cluster systems as catalyst precursors: the role of the chelating effect"**

**Description**

This invention relates to a process for the catalytic reduction of nitroaromatic compounds.

More particularly, the present invention relates to a catalytic process for reducing nitroaromatic compounds through displacement of hydrogen from a carbon monoxide and water system or from synthesis gas in the presence of complexes of rhodium, iridium, ruthenium or osmium.

The resulting compounds consist of arylamines in general.

The products obtained are usefully employed in a wide range of industrial applications. In fact, they represent active intermediates for orgnaic synthesis in general, with particular possibilities in the pure chemicals field. Among said products, aniline is utliized in the field of synthetic rubbers, in the production of isocyanates (polyurethans), and especially, along with other arylamines prepared according to this invention, in the field relating to the dyestuff and intermediate industry, in photographic and pharmaceutical products, in plastic materials, propellants, insecticides and the like.

Methods are known for catalytic reduction of aromatic nitroderivatives by employing, as a hydrogenation source, both molecular hydrogen and hydrogen-donor, generally carbon monoxide and water, alcohols, etc.

In particular, in the article of Kaneda and Al published in J. Molecular Catalysis 12 (1981) p. 385—387 (see C.A.95.192961v) a method has been described for reducing aromatic nitroderivatives, e.g. nitrobenzene to aniline, through displacement of hydrogen from the carbon monoxide and water system or from synthesis gas catalyzed by cluster or carbonyl compounds of Rh, Ru and Fe such as, for example $Rh_6(Co)_{16}$ in the presence of various amines such as triethylamine, N,N-dimethylbenzylamine, pyridine, N-methylpyrrolidine, N,N,N',N',tetramethylethylene diamine, para-dimethyl-aminopyridine, among which pyridine and N,N,N',N'-tetramethyl-ethylenediamine have proved to be the most active.

In these methods the amine/metal ratios used for the reaction have to be exceptionally high to obtain a valuable conversion percentage.

Amines are generally used at concentrations considerably higher than the concentration of the substrate (aromatic nitroderivative).

A catalyst system has been now determined, which consists of complexes of Rh, Ir, Ru and Os in the presence of, or containing, aromatic bidentate or tridentate nirogen chelants never used until now, which unexpectedly provide a catalytic activity far higher than the hydrogen-displacement catalyst of the prior art.

It is thus an object of the present invention to provide a method for the catalytic reduction of nitroaromatic compounds to aromatic amines, with complexes of Rh, Ir, Ru and Os, in the presence of, or containing; aromatic, bidentate or tridentate nitrogen chelants, such method being simple, economic and particularly selective and especially directed to the obtention of a high catalysis rate while employing the $CO + H_2O$ system or the $(CO + H_2) + H_2O$ system as a hydrogen-donor.

FR—A—2 450 798 discloses a process directed to the catalytic reduction of carbonyl compounds (ketones or aldehydes) to obtain the corresponding alcohol compounds, by the hydrogen transfer reaction from alcohols to such carbonyl compounds.

The hydrogen transfer reaction from alcohols is carried out in the presence of a catalytic system which comprises, further the Rh or Ir complex salts, also co-catalytic amounts of a mineral or organic base, in essentially anhydrous conditions and under a nitrogen atmosphere. Further, the Rh and Ir complexes therein disclosed do not contain CO groups as a ligand for the transition metal Rh and Ir.

There is dealt, therefore, with a quite different reaction as to its chemistry grounds as directed to a transfer reaction of hydrogen from alcohols carried out in the substantial absence of water and, as to the catalyst system adopted, comprising different Rh and Ir complexes and co-catalysts bases. No enabling suggestion can be found in such a disclosure as to the possibility of reducing nitro-aromatic compounds by displacement of hydrogen from a $CO + H_2O$ peculiar system.

This and other objects, which will appear more clearly to those skilled in the art from the description herein-under, are achieved, according to the present invention, by a process for the catalytic reduction of nitroaromatic compounds through displacement of hydrogen from carbon monoxide and water or from synthesis gas to nitroaromatic compounds, said reduction being catalyzed by complexes of rhodium, iridium, ruthenium and osmium, such process being characterized in that the $CO + H_2O$ system or the $(CO + H_2) + H_2O$ system is made to react with a nitroaromatic compound of formula:

$$Ar(NO_2)_x \qquad (I)$$

wherein:

Ar is an aryl group or a hetero-aryl group, possibly substituted by inert groups;

x is an integer from 1 to 3,

in the presence of a complex catalyst of formula:

$$\{M\ Chel\ (L)_2\}^+X^- \qquad (II)$$

or of a catalyst composed by carbonyl compounds and chelants of formula:

$$Mz\,(CO)_y + n\ Chel \qquad (III)$$

wherein:

M = Rh, Ir, Ru, Os;
"Chel" represents a bidentate or tridentate aromatic nitrogen chelating compound;
"L" represents a molecule of carbon monoxide or of an olefin or half a molecule of a diolefin;
$X^-$ is an anion selected from among $Cl^-$, $Br^-$, $I^-$, $PF_6^-$, $BF_4^-$, $B(C_6H_5)_4^-$, $CO_3^-$ and $HCO_3^-$;
z is an integer comprised between 1 and 20;
y is an integer comprised between 4 and 30;
n is an integer or a fractional number comprised between 0,1 and 20, at temperatures ranging from about 25°C to 250°C and at carbon monoxide pressure ranging from 1 to 150 atmospheres.

As cited herein-above, Ar represents in particular an aryl group or a hetero-aryl group, optionally condensed, having at least 5 atoms in the carbon ring (aryl groups), or optionally with hetero-atoms, selected from N, S and O (hetero-aryl groups), such as for example the phenyl, naphthalene, anthracene, thienyl, furan, pyridine, quinoline groups.

Furthermore, the Ar group as defined herein-above can also include inert substituents in the rection conditions, selected e.g. from the alkyl, alkoxide, aminic groups.

Thus, derivatives belonging to the classes of benzene, naphthalene, anthracene, thienyl, furan, pyridine, quinoline can be used as starting nitroaromatic derivatives.

The reaction is accomplished in solvents such as $H_2O$, ethanol, methanol, tetrahydrofuran, dioxane in admixture with water.

The hydrogen displacement reaction can be schematically represented by the following equation:

$$Ar{-}NO_2 + 3CO + H_2O \xrightarrow[\text{solv.}]{\text{cat.}} Ar{-}NH_2 + 3CO_2$$

In general:

$$Ar(NO_2)_x + X(3CO) + XH_2O \xrightarrow[\text{solv.}]{\text{cat.}} Ar(NH_2)_x + X(3CO_2)$$

As regards the catalysts, the systems employed according to the invention have formulas (II) and (III) in which the aromatic bidentate or tridentate nitrogen chelant is preferably selected from among 2,2'-bipyridyl (bipy),4,4-dimethyl-2,2'-bipyridyl, 1,10-phenanthroline, 5,6-dimethyl-1,10-phenanthroline, 2,9-dimethyl-1,10-phenanthroline, 4,7-dimethyl-1,10-phenanthroline, 3,4,7,8-tetramethyl-1,10-phenanthroline, 3,4,5,6,7,8-hexamethylphenanthroline, 2,3,4,7,8,9-hexamethylphenanthroline, 2,4,7,9-tetramethyl-phenanthroline, sulphonated phenanthrolines, N,N,N',N'-ortho-phenylendiamine, N,N,N',N'-1,8-diamino-naphthalene and terpyridyl.

As catalysts according to the present invention it is possible to use, for example:

$$\{Rh\ 3,4,7,8(CH_3)_4\ phen\ (CO_2\}B(C_6H_5)_4$$

$$\{Rh\ 4,7(CH_3)_2\ phen(CO)_2\}B(C_6H_5)_4$$

$$\{Rh\ phen\ (CO_2\}B(C_6H_5)_4$$

$Rh_6(CO)_{16}$ + n Chel (n = 60)
$Ru_3(CO)_{12}$ + 3 (bipy), $Ru_3(CO)_{12}$ + terpy,
$Ru_3(CO)_{12}$ + 3 (phen)
$Ru_3(CO)_{12}$ + 1,5 phen
$Ru_3(CO)_{12}$ + 3 (3,4,7,8 $(CH_2)_4$ phen).

wherein:

"phen" means 1,10-phenanthroline,
"bipy" means 2,2'-bipyridyl,
"terpy" means terpyridyl.
The following have proved to be particularly effective:
$Rh_6(CO)_{16}$ + 60 (3,4,7,8$(CH_3)_4$ phen) and
$Ru_3(CO)_{12}$ + 3 phen
$Ru_3(CO)_{12}$ + 1,5 phen.

The complexes used as catalysts according to the present invention are prepared, in turn, by means of known and conventional techniques.

For example, the rhodium complex of formula:

$$\{Rh\ 3,4,7,8(CH_3)_4\ phen\ (CO)_2\}B(C_6H_5)_4$$

can be prepared starting from:

$$\{Rh\ 3,4,7,8(CH_3)_4\ phen\ COD\}B(C_6H_5)_4;$$

(COD = 1,5-cyclooctadiene) by treatment with carbon monoxide in methanol at room temperature and pressure.

The carbonyl clusters are also prepared according to conventional techniques.

The catalyst is employed, according to the invention, in amounts which may be comprised within a wide range.

Advantageous results are obtained by using, for each mole of nitroaromatic compound, amounts ranging from $1 \times 10^{-2}$ to $1 \times 10^{-5}$ grams atoms of metal contained in the catalyst.

Suitable reaction mediums are ethanol —$H_2O$ (5%), tetrahydrofuran-water.

The reducing reaction according to the invention is carried out at carbon monoxide pressures ranging from atmospheric pressure to 150 atmospheres.

Prevailing temperatures are in the range of from 25°C to about 200°C.

Reducible nitroaromatic compounds according to the present invention are, in particular, among the aromatics, nitrobenzene, nitrotoluene, nitroanisole, nitrochlorobenzene, nitrobenzonitrile, nitronaphthalene, dinitrobenzene, dinitrotoluene, dinitroaniline, dinitrobenzamide; amont the heteromatics, the nitropyridines, nitroquinolines, etc.

The practice is then separated according to conventional techniques. In practice, it is a matter of separating the solvent, if any, by distillation, while the high-boiling portion consists of the compound generally quantitatively hydrogenated.

In particular, the catalyst can be removed from the reaction medium by adsorption on animal charcoal, or ion-exchange resins (in particular in the case of the sulphonated phenanthrolines).

According to a preferred embodiment, operating is carried out as follows.

A solution containing the catalyst and the nitroatomatic compound is introduced, in a carbon monoxide atmosphere, into a reactor equipped with feeding systems for the reagents, thermoregulated and magnetically stirred. The desired amount of carbon monoxide under pressure is then introduced, whereupon it is heated to the prefixed temperature and for the prefixed period of time. At the conclusion of the reaction, which is controlled, for example, by gaschromatography, the product is isolated according to conventional techniques.

The process according to the present invention permits to obtain throuigh a catalytic activity far higher than that of the hydrogen displacement catalysts of the art, high conversion yields exeeding 98% in shorter times, employing chelant concentrations which are considerably lower than the concentrations of the substrate and with a high substrate/catalyst ratio.

The process according to the present invention, permits an easier isolation of the reaction products since it operates in the absence of co-catalysts, which are usually employed in the art.

The invention will now be further described in the following examples, which are however given for mererly illustrative purposes, examples 43 through 52 being given by way of comparison with the processes of the prior art.

The symbols employed in the examples are:

"bipy", which means 2,2'-bipyridyl,

"phen", which means 1,10-phenanthroline,

S = substrate,

"chel", which means a chelating compound as is defined in the description. The ratios are expressed in moles.

## Example 1

To 50 ml of ethanol-$H_2O$ (5%) were added 0.615 g ($5 \times 10^{-3}$ moles) of nitrobenzene and then $5 \times 10^{-3}$ m.moles of $\{Rh\ 3,4,7,8(CH_3)_4\ phen\ (CO)_2\}B(C_6H_5)_4$.

The resulting solution was poured into a 100 ml capacity autoclave.

The autoclave was charged with 30 atmospheres of carbon monoxide at room temperature and successively was heated to 165°C. The reaction trend was checked by means of gas chromatography taking samples at intervals. After 2 hours, aniline with a yield of 43% with respect to the starting nitrobenzene was obtained. Ratios: S/Rh = 1,000; Chel/Rh = 1.

## Example 2

To 50 ml of ethanol-$H_2O$ (5%) were added 0.615 g ($5 \times 10^{-3}$ moles) of nitrobenzene and then $5 \times 10^{-3}$ m.moles (1.18 mg) of $3,4,7,8(CH_3)_4$ phen.

The solution thus obtained was poured into a 100 ml capacity autoclave containing 0.83 × $10^{-3}$ m.moles (0.88 mg) of $Rh_6(CO)_{16}$.

The autoclave was charged with 30 atmospheres of carbon monoxide at room temperature and successively it was heated to 165°C.

After 2 hours, aniline with a yield of 43% with respect to the starting nitrobenzene was obtained. Ratios: S/Rh = 1,000; Chel/Rh = 1.

Example 3

To 50 ml of ethanol-$H_2O$ (5%) were added 0.615 g (5 × $10^{-3}$ moles) of nitrobenzene, then 3.75 mg (5 × $10^{-3}$ m.moles) of {Rh 3,4,7,8$(CH_3)_4$ phen $(CO)_2$}$B(C_6H_5)_4$ and finally 3.54 mg (1.5 × $10^{-2}$ m.moles) of 3,4,7,8-$(CH_3)_4$ phen.

The resulting solution was poured into a 100 ml capacity autoclave.

The autoclave was charged with 30 atmospheres of carbon monoxide at room temperature and successively heated to 165°C.

After 2 hours, aniline with a yield of 96% in respect of the starting nitrobenzene was obtained. Ratios: S/Rh = 1,000; Chel/Rh = 4.

Example 4

To 50 ml of ethanol-$H_2O$ (5%) were added 0.615 g (5 × $10^{-3}$ moles) of nitrobenzene and successively 4.72 mg of 3,4,7,8-$(CH_3)_4$ phen. The solution thus obtained was poured into a 100 ml capacity autoclave containing 0.88 mg (0.83 × $10^{-3}$ m.moles) of $Rh_6(CO)_{16}$.

30 atmospheres of carbon monoxide at room temperature were charged into the autoclave, which was then heated to 165°C. After 2 hours, aniline with a yield of 96% in respect of the starting nitrobenzene was obtained. Ratios: S/Rh = 1,000; Chel/Rh = 4.

Example 5

Operating took place as in example 1, employing 2.36 mg of 3,4,7,8-$(CH_3)_4$ phen.

After 2 hours, aniline with a yield of 62% was obtained. Ratios: S/Rh = 1,000; Chel/Rh = 2.

Example 6

Operating took place as in example 4, employing 11.8 mg of 3,4,7,8-$(CH_3)_4$ phen.

After 2 hours, aniline with a yield of 100% was obtained. Ratios: S/Rh = 1,000; Chel/Rh = 10.

Example 7

Operating took place as in example 2, employing 0.78 mg (5 × $10^{-3}$ m.moles) of bipy.

After 2 hours, aniline with a yield of 0.82% referred to the starting nitrobenzene was obtained. Ratios: S/Rh = 1,000; Chel/Rh = 1.

Example 8

Operating took place as in example 2, employing 0.96 mg (5 × $10^{-3}$ m.moles) of phen.

After 2 hours, aniline with a yield of 7.5% in respect of the starting nitrobenzene was obtained. Ratios: S/Rh = 1,000; Chel/Rh = 1.

Example 9

Operating took place as in example 2, employing 9.6 mg (5 × $10^{-2}$ m.moles) of phen.

After 2 hours, aniline with a yield of 54% calculated on the starting nitrobenzene was obtained.

After 3 hours, aniline with a yield of 91% calculated on the starting nitrobenzene was obtained. Ratios: S/Rh = 1,000; Chel/Rh = 10.

Example 10

Operating took place as in example 2, employing 1.04 mg (5 × $10^{-3}$ moles) of 4,7$(CH_3)_2$ of phen.

After 2 hours, aniline with a yield of 25% with respect to the starting nitrobenzene was obtained. Ratios: S/Rh = 1,000; Chel/Rh = 1.

Example 11

Operating took place as in example 2, employing 4.16 mg (2 × $10^{-2}$ m.moles) of 4,7 $(CH_3)_2$ phen.

After 2 hours, aniline with a yield of 64.5% with respect to the starting nitrobenzene was obtained. Ratios: S/Rh + 1,000; Chel/Rh = 4.

Example 12

Operating took place as in example 2, employing 1.80 mg (5 × $10^{-3}$ m.moles) of 2.9 $(CH_3)_2$ 4,7 $(C_6H_5)_2$ phen.

After 2 hours, aniline with a yield of 4.5% with respect to the starting nitrobenzene was obtained. Ratios: S/Rh = 1,000; Chel/Rh = 1.

Example 13

Operating took place as in example 2, employing 10.73 mg ($2 \times 10^{-2}$ m.moles) of sulphonated phen of formula:

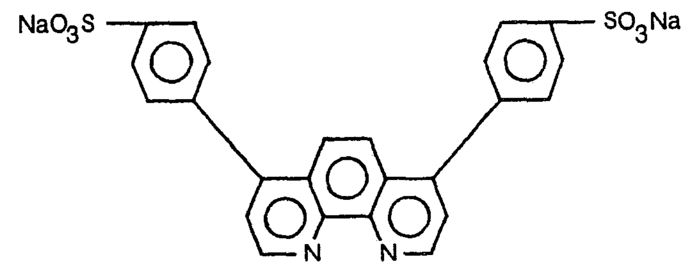

After 2 hours, 45.5% of aniline, calculated on the starting nitrobenzene, was obtained. Ratios: S/Rh = 1,000; Chel/Rh = 4.

Example 14

Operating took place as in example 13, employing ethanol-$H_2O$ (10%).

After 2 hours, aniline with a yield of 59.5% referred to the starting nitrobenzene was obtained. Ratios: S/Rh = 1,000; Chel/Rh = 4.

Example 15

Operating took place as in example 14, employing 2.68 mg ($5 \times 10^{-3}$ m.moles) of sulphonated phen.

After 2 hours, 20% of aniline calculated on the starting nitrobenzene was obtained. Ratios: S/Rh = 1,000; Chel/Rh = 1.

Example 16

Operating took place as in example 14, employing 11.33 mg ($2 \times 10^{-2}$ m.moles) of 2,9 dm sulpohonated phen of formula:

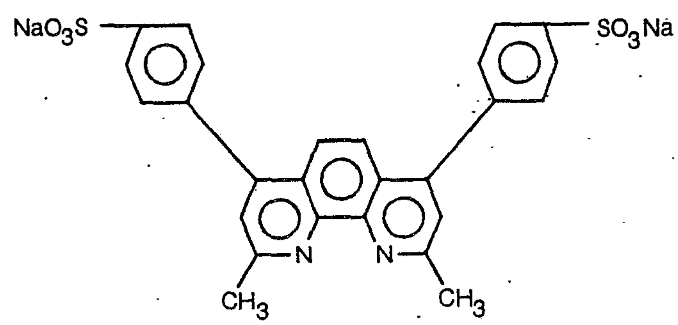

After 2 hours, aniline with a yield of 7% calculated on the starting nitrobenzene was obtained. Ratios: S/Rh = 1,000; Chel/Rh = 4.

Example 17

To 50 ml of ethanol-$H_2O$ (5%) were added 0.615 g ($5 \times 10^{-3}$ moles) of nitrobenzene and thereafter 0.96 mg ($5 \times 10^{-3}$ m.moles) of phen.

The resulting solution was poured into a 100 ml capacity autoclave containing 1.06 mg ($1.6 \times 10^{-3}$ m.moles) of $Ru_3(CO)_{12}$.

30 atmospheres of carbon monoxide at room temperature were charged into the autoclave, which was then heated to 165°C.

After 2 hours, aniline with a yield higher than 98% calculated on the starting nitrobenzene was obtained. Ratios: R/Ru = 1,000; Chel/Ru = 1.

Example 18

Operating took place as in example 17, employine 0.48 mg (2,5 $\times 10^{-3}$ moles) of 1,10-phenanthroline.

After 2 hours, aniline with a yield higher than 98% calculated on the starting nitrobenzene was obtained. Ratios: S/Ru = 1,000; Chel/Ru = 0.5.

Example 19

Operating took place as in example 17, employing 1.18 mg ($5 \times 10^{-3}$ m.moles) of 3,4,7,8-$(CH_3)_4$ phen.

After 2 hours, aniline with a yield higher than 97% calculated on the starting nitrobenzene was obtained. Ratios: S/Ru = 2,000; Chel/Ru = 1.

Example 20

Operating took place as in example 17, employing 1.92 mg ($1 \times 10^{-2}$ m.moles) of phen.

After 2 hours, aniline with a yield of 56.5% referred to the starting nitrobenzene was obtained. Ratios: S/Ru = 1,000; Chel/Ru = 2.

Example 21

Operating took place as in example 17, employing 0.78 mg ($5 \times 10^{-3}$ m.moles) of bipy.

After 2 hours, aniline with a yield of 79% in respect of the starting nitrobenzene was obtained. Ratios: S/Ru = 1,000; Chel/Ru = 1.

Example 22

Operating took place as in example 17, employing 0.685 g ($5 \times 10^{-3}$ moles) of paranitrotoluene.

After 2 hours, paratoluidine with a yield of 98.5% referred to the starting paranitrotoluene was obtained. Ratios: S/Ru = 1,000; Chel/Ru = 1.

Example 23

Operating took place as in example 17, employing 0.765 g ($5 \times 10^{-3}$ moles) of paranitroanisole.

After 2 hours, paramethoxyaniline with a yield of 66% on the starting paranitroanisole was obtained. Ratios: S/Ru = 1,000; Chel/Ru = 1.

Example 24

Operating took place as in example 17, employing 0.787 g of p-chloronitrobenzene ($5 \times 10^{-3}$ moles).

After 2 hours, p-chloroaniline with a yield of 7% on the starting nitroderivative was obtained. Ratios: S/Ru = 1,000; Chel/Ru = 1.

Example 25

Operating took place as in example 17, employing 0.910 ($5 \times 10^{-3}$ moles) of 2,4-dinitro-toluene and 3.84 mg ($2 \times 10^{-2}$ m.moles) of phen as well as 4.24 mg ($6.67 \times 10^{-3}$ m.moles) of $Ru_3(CO)_{12}$.

After 15 hours, 2,4-diamino-toluene with a yield of 77% with respect to the starting 2,4-dinitro-toluene was obtained. Ratios: S/Ru = 250; Chel/Ru = 1.

Example 26

Operating took place as in example 17, employing 1.92 mg of phen ($1 \times 10^{-2}$ m.moles) and 2.12 mg ($3.34 \times 10^{-3}$ m.moles) of $Ru_3(CO)_{12}$.

After 90 minutes, aniline with a yield higher than 98% with respect to the starting nitrobenzene was obtained. Ratios: S/Ru = 500; Chel/Ru = 1.

Example 27

Operating took place as in example 17, employing 3.84 mg of phen ($2 \times 10^{-2}$ m.moles) and 4.24 mg ($6.64 \times 10^{-3}$ m.moles) of $Ru_3(CO)_{12}$.

After 60 minutes, aniline with a yield of 98% with respect to the starting nitrobenzene was obtained. Ratios: S/Ru = 250; Chel/Ru = 1.

Example 28

Operating took place as in example 27, working at a temperature of 125°C.

After 4 hours, aniline with a 96% yield with respect to the starting nitrobenzene was obtained. Ratios: S/Ru = 250; Chel/Ru = 1.

Example 29

Operating took place as in example 27, working at 90°C.

After 4 hours, aniline with a 10.5% yield with respect to the starting nitrobenzene was obtained. Ratios: S/Ru = 250; Chel/Ru = 1.

Example 30

Operating took place as in example 27, working at 50°C.

After 17 hours, aniline with a 0.5% yield with respect to the starting nitrobenzene was obtained. Ratios: S/Ru = 250; Chel/Ru = 1.

Example 31

Operating took place as in example 17, employing a carbon monoxide pressure of 50 atmospheres (at room temperature).

After 2 hours, aniline with a 98% yield with respect to the starting nitrobenzene was obtained. Ratios: S/Ru = 1,000; Chel/Ru = 1.

Example 32

Operating took place as in example 17, employing 10 atmospheres of carbon monoxide (at room temperature).

After 2 hours, aniline with a 90% yield with respect to the starting nitrobenzene was obtained. Ratios: S/Ru = 1,000; Chel/Ru = 1.

Example 33

Operating took place as in example 17, employing 5 atmospheres of carbon monoxide (at room temperature).

After 16 hours, aniline with a 90% yield in respect of the starting nitrobenzene was obtained. Ratios: S/Ru = 1,000; Chel/Ru = 1.

Example 34

Operating took place as in example 17, employing 1.23 g ($1 \times 10^{-2}$ moles) of nitrobenzene and a CO pressure of 50 atmospheres.

After 2 hours, aniline with a 93% yield in respect of the starting nitrobenzene was obtained. Ratios: S/Ru = 2,000; Chel/Ru = 1.

Example 35

Operating took place as in example 17, employing 2.46 g ($2 \times 10^{-2}$ moles) of nitrobenzene and a CO pressure of 50 atmospheres.

After 4 hours, aniliine with a 35.5% yield with respect to the starting nitrobenzene was obtained. Ratios: S/Ru = 4,000; Chel/Ru = 1.

Example 36

Operating took place as in example 28, while employing 0.38 mg of phen.

After 2 hours, aniline with a yield of 25.6% referred to the starting nitrobenzene, was obtained. Ratios: S/Ru = 250; Chel/Ru = 0.1.

Example 37

Operating took place as in example 28, employing 0.47 mg of 3,4,7,8-$(CH_3)_4$ phen.

After 2 hours, aniline with a yield of 21.3% referred to the starting nitrobenzene, was obtained. Ratios: S/Ru = 250; Chel/Ru = 1.

Example 38

Operating took place as in example 28, employing 12.48 mg of 2,9 $(CH_3)_2$ phen.

After 3 hours, aniline with a 90%-yield in respect of the starting nitrobenzene was obtained. Ratios: S/Ru = 250; Chel/Rh = 3.

Example 39

To 50 ml of ethanol-$H_2O$ (5%) were additioned: 0.615 g ($5 \times 10^{-3}$ moles) of nitrobenzene and, successively, 4.02 mg {Ir 3,4,7,9 $Me_4$ phen$(CO)_2$}$B(C_6H_5)_4$.

The solution thus obtained was poured into a 100 ml capacity autoclave. Into the autoclave, 30 atmospheres of carbon monoxide were charged at room temperature, and there after heated to 165°C.

After 2 hours, aniline with a 1.3% yield referred to the starting nitrobenzene was obtained. Ratio: S/Ir = 1,000.

Example 40

Operating took place as in example 17, employing 1.5 mg of $Os_3(CO)_{12}$.

After 2 hours, aniline with a yield of 1%, with respect to the starting nitrobenzene, was obtained. Ratios: S/Os = 1,000; Chel/Os = 1.

Example 41

Operating took place as in example 2, employing 1.16 mg of terpyridyl ($5 \times 10^{-3}$ m.moles).

After 2 hours, aniline with a yield of 4.5% calculated on the starting nitrobenzene was obtained. Ratios: S/Rh = 1,000; Chel/Rh = 1.

Example 42

Operating took place as in example 17, employing 1.16 mg of terpyridyl ($5 \times 10^{-3}$ m.moles).

After 2 hours, aniline with a yield of 90%, referred to the starting nitrobenzene, was obtained. Ratios: S/Ru = 1,000; Chel/Ru = 1.

Examples 43—46 (comparative tests)

A set of 4 nitrobenzole reduction tests was carried out under the same conditions as in example 2, one of such tests not employing 3,4,7,8-$(CH_3)_4$phen and the other three tests employing, instead of 3,4,7,8-

$(CH_3)_4$phen, different amounts of pyridine, which is one of the amines capable of imparting, according to the prior art, the highest catalytic activity. The reaction conditions employed for the synthesis were as follows: catalyst $Rh_6(CO)_{16}$ ± amines; T = 165°C; CO pressure = 30 atmospheres; {Rh} = 1 × $10^{-4}$ moles; S/Rh = 1,000; sovlent = ethanol-$H_2O$ (5%).

The various amounts of pyridine employed and the results obtained are recorded on Table I in comparison with example 2 of the present invention.

Examples 47—49 (comparative tests)

A series of 3 nitrobenzole reduction tests was carried out under the same reaction conditions as in examples 43—46, using the $Rh_6(CO)_{16}$ + amine catalytic system, and employing, as an amine, N,N,N',N',tetramethylethylene diamine in different amounts, said amine being capable of imparting, according to the prior art, the highest catalytic activity.

The employed diamine amounts and the results obtained recorded on Table II in comparison with example 5 of the present invention.

TABLE I

| Example n° | Amine | Amine/Rh | % Conversion (2 hours) |
|---|---|---|---|
| 43 | — | — | 0 |
| 44 | pyridine | 2 | 0 |
| 45 | pyridine | 200 | traces |
| 46 | pyridine | 2.000 | 2.4 |
| 2 | 3,4,7,9-$(CH_3)_4$ phen | 1 | 43 |

TABLE II

| Example n° | Amine | Amine/Rh | % Conversion (2 hours) |
|---|---|---|---|
| 47 | N,N,N',N'-tetramethylethylene diamine | 10 | 6.5 |
| 48 | N,N,N',N',tetramethylethylene diamine | 100 | 26.6 |
| 49 | N,N,N',N',tetramethylethylene diamine | 500 | 56.3 |
| 5 | 3,4,7,9-$(CH_3)_4$ phen | 2 | 62 |

Examples 50—52 (comparative tests)

Nitrobenzole to aniline reduction tests were carried out with the CO + $H_2O$ system, under the same reaction conditions of examples 43—46, by employing the $Ru_3(CO)_{12}$ catalytic system in the absence and in the presence of various amounts of pyridine.

The obtained results are recorded on Table III in comparison with examples 17, 18 and 19 of the present invention.

TABLE III

| Example N° | Amine | Amine/Ru | % Conversion (2 hours) |
|---|---|---|---|
| 50 | — | — | 8 |
| 51 | pyridine | 2 | 9.3 |
| 52 | pyridine | 20 | 10 |
| 17 | 1,10-phenanthroline | 1 | 98 |
| 18 | 1,10-phenanthroline | 0.5 | 98 |
| 19 | 3,4,7,8$(CH_3)_4$phen | 1 | 97 |

**Claims**

1. A process for the catalytic reduction of nitroaromatic compounds by displacement of hydrogen from carbon monoxide and water or from synthesis gas to nitroaromatic compounds, such reduction being catalyzed by complexes of rhodium, iridium, ruthenium and osmium; characterized in that the $CO + H_2O$ system or the $(CO + H_2) + H_2O$ system is reacted with a nitroaromatic compound of formula:

$$Ar\text{-}(NO_2)_x \qquad (I)$$

wherein:
Ar is an aryl group or a hetero-aryl group, optionally substituted by inert groups;
x is an integer selected from 1 to 3,
in the presence of a complex catalyst having the formula:

$$\{M\ Chel\ (L)_2\}^+X^- \qquad (II)$$

or of a catalyst composed by carbonyl compounds and chelants of formula:

$$Mz\ (CO)_y + n\ Chel \qquad (III)$$

wherein:
M = Rh, Ir, Ru, Os;
"Chel" is a chelating bidentate or tridentate aromatic nitrogen compound:
"L" is a molecule of carbon monoxide or of an olefin or half a molecule of a diolefin;
$X^-$ is an anion selected from among $Cl^-$, $Br^-$, $I^-$, $PF_6^-$, $BF_4^-$, $B(C_6H_5)_4^-$, $CO_3^{--}$ and $HCO_3^-$;
z is an integer comprised between 1 and 20;
y is an integer comprised between 4 and 30;
n is an integer or a fractional number comprised beween 0,1 and 20, at temperatures ranging from about 25°C to 250°C, at a carbon monoxide pressure ranging from 1 to 150 atmospheres.

2. The process according to claim 1, characterized in that the nitroaromatic compound of formula (I) is selected from the derivatives of the class of benzene, naphthalene, anthracene, thienyl, furan, pyridine and quinoline, optionally substituted by inert groups, under the reaction conditions, such as the alkyl, alkoxyl, aminic groups and the like.

3. The process according to claim 1, characterized in that it is carried out in a medium selected from among water, ethanol, methanol; tetrahydrofuran, dioxane in admixture with water.

4. The process according to claim 1, characterized in that the chelating nitrogen compound "Chel" contained in the catalysts of formula (II) and (III) is selected from 2,2'-dipyridyl, 4,4'-dimethyl-2,2'-dipyridyl, 1,10-phenanthroline, 5,6-dimethyl-1,10-phenanthroline, 2,9-dimethyl-1,10-phenanthroline, 4,7-dimethyl-1,10-phenanthroline, 3,4,7,8-tetra-methyl-1,10-phenanthroline, 3,4,5,6,7,8-hexamethyl-phenanthroline, 2,3,4,7,8,9-hexamethyl-phenanthroline, 2,4,7,9-tetramethyl-phenanthroline, sulphonated phenanthrolines, N,N,N',N'-ortho-phenylene diamine and N,N,N',N'-1,8-diaminonaphthalene and terpyridyl.

5. The process according to claim 1, characterized in that for 1 mole of nitroaromatic compound (I) there are employed from $1 \times 10^{-2}$ to $1 \times 10^{-5}$ grams atoms of metal contained in the catalyst.

6. The process according to claim 1, characterized in that the nitroaromatic compound (I) is selected from nitrobenzene, nitrotoluene, nitroanisole, nitrochlorobenzene, nitroaniline, nitrobenzoamide, nitrobenzonitrile, nitronaphthalene, dinitrobenzene, dinitrotoluene, dinitroaniline, dinitrobenzoamide, nitropyridine, nitroquinoline.

7. The process according to claim 1, characterized in that the catalyst of formula (II) or (III) is selected from amongst:
$\{Rh\ 3,4,7,8\text{-}(CH_3)_4\ phen(CO)_2\}B(C_6H_5)_4$
$\{Rh\ 4,7(CH_3)_2\ phen(CO)_2\}B(C_6H_5)_4$
$\{Rh\ phen(CO)_2\}B(C_6H_5)_4$
$Rh_6(CO)_{16} + 60\ Chel$
$Rh_6(CO)_{16} + 60\ \{3,4,7,8\text{-}(CH_3)_4\ phen\}$
$Ru_3(CO)_{12} + 3\ bipy$, $Ru_3(CO)_{12} + terpyridyl$
$Ru_3(CO)_{12} + 3\ phen$, $Ru_3(CO)_{12} + 1,5\ phen$
$Ru_3(CO)_{12} + 3\ \{3,4,7,8\text{-}(CH_3)_4\ phen\}$
wherein:
"phen" means phenanthroline,
"Chel" has the meaning as defined in claims 1 and 4,
"bipy" means 2,2'-dipyridyl.

## Patentansprüche

1. Verfahren zum katalytischen Reduzieren nitroaromatischer Verbindungen durch Überführung von Wasserstoff aus Kohlenoxyd und Wasser oder synthetischem Gas in nitroaromatische Verbindungen, wobei diese Reduktion durch Rhodium:, Iridium= und Osmiumkomplexe katalysiert wird, dadurch gekennzeichnet, dass man das System $CO + H_2O$ oder das System $(CO + H_2) + H_2O$ mit einer nitroaromatischen Verbindung der Formel:

$$Ar—(NO_2)_x \qquad (I)$$

worin:

Ar eine gegebenenfalls durch inerte Gruppen substituierte Arylgruppe oder Heteroarylgruppe ist;
x eine ganze Zahl von 1 bis 3 darstellt;

in Anwesenheit eines komplexen Katalysators der Formel:

$$(M\ Chel\ (L)_2)^+X^- \qquad (II)$$

oder eines aus Carbonylverbindungen und chelatbildnern bestehenden Katalysators der Formel:

$$Mz\ (CO)_y + n\ Chel \qquad (III)$$

reagieren lässt, wo:

M = Rh, Ir, Ru, Os;
Chel eine chelatbildende aromatische Bildentat= oder Tridentatverbindung ist;
L ein Kohlenoxyd= oder Olefinmolekül oder ein halbes Diolefinmolekül ist;
$X^-$ ein Anion der Gruppe $Cl^-$, $Br^-$, $I^-$, $PF_6^-$, $BF_4^-$, $B(C_6H_5)_4^-$, $CO_3^{--}$ und $HCO_3^-$ ist;
z eine ganze Zahl von 4 bis 20 ist;
y eine ganze Zahl von 4 bis 30 ist;
n eine ganze Zahl oder eine Bruchzahl von 0,1 bis 20 ist;

wobei diese Reaktion bei Temperaturen von etwa 25°C bis 250°C und bei einem Kohlenoxyddruck von 1 bis 150 atm durchgeführt wird.

2. Verfahren nach Anspruch 1, dadurch gekennzeichnet, dass die nitroaromatische Verbindung der Formel (I) unte den Derivaten der Benzol, Naphtalin, Anthracen, Thienyl, Furan, Pyridin und Chinolin umfassenden Stoffklasse gewählt wird, gegebenenfalls mit Substitution durch inerte Gruppen unter den Reaktionsbedingungen, beispielsweise durch Alkyl=, Alkoxyl=, Amin= oder dergleichen Gruppen.

3. Verfahren nach Anspruch 1, dadurch gekennzeichnet, dass es in einem Medium der Gruppe Wasser, Äthanol, Methanol, Tetrahydrofuran, Dioxan, mit Wasser gemischt, durchgeführt wird.

4. Verfahren nach Anspruch 1, dadurch gekennzeichnet, dass die chelatbildende Stickstoffverbindung "Chel" der Katalysatoren in Formel (II) und (III) unter den Stoffen folgender Gruppe gewählt wird: 2,2'-Dipyridyl, 4,4'-Dimethyl-2,2'-dipyridyl, 1,10-Phenanthrolin, 5,6-Dimethyl-1,10-phenanthrolin, 2,9-Dimethyl-1,10-phenanthrolin, 4,7-Dimethyl-1,10-phenanthrolin, 3,4,7,8-Tetramethyl-1,10-phenanthrolin, 3,4,5,6,7,8-Hexamethylphenanthrolin, 2,3,4,7,8,9-Hexamethylphenanthrolin, 2,4,7,9-Tetramethylphenanthrolin, Phenanthrolinsulfonate, N,N,N',N',Orthophenylendiamin und N,N,N',N'-1,8-Diaminonaphtalin und Terpyridyl.

5. Verfahren nach Anspruch 1, dadurch gekennzeichnet, man pro Mol der nitroaromatischen Verbindung (I) $1 \times 10^{-2}$ bis $1 \times 10^{-5}$ Gramm der im Katalysator enthaltenen Metall atome einsetzt.

6. Verfahren nach Anspruch 1, dadurch gekennzeichnet, dass die nitroaromatische Verbindung (I) unter den Stoffen folgender Gruppe gewählt wird: Nitrobenzol, Nitrotoluol, Nitroanisol, Nitrochlorobenzol, Nitroanilin, Nitrobenzoamid, Nitrobenzonitril, Nitronaphtalin, Dinitrobenzol, Dinitrotoluol, Dinitroanilin, Dinitrobenzoamid, Nitropyridin, Nitrochinolin.

7. Verfahren nach Anspruch 1, dadurch gekennzeichnet, dass der Katalysator der Formel (I) oder (III) unter den Stoffen folgender Gruppe gewählt wird:

$\{Rh\ 3,4,7,8(CH_3)_4\ phen(CO)_2\}B(C_6H_5)_4$
$\{Rh\ 4,7(CH_3)_2\ phen(CO)_2\}B(C_6H_5)_4$
$\{Rh\ phen(CO)_2\}B(C_6H_5)_4$
$Rh_6(CO)_{16} + 60$ Chel
$Rh_6(CO)_{16} + 60\ \{3,4,7,8\text{-}(CH_3)_4\ phen\}$
$Ru_3(CO)_{12} + 3$ bipy, $Ru_3(CO)_{12}$ + terpyridyl
$Ru_3(CO)_{12} + 3$ phen, $Ru_3(CO)_{12} + 1,5$ phen
$Ru_3(CO)_{12} + 3\ \{3,4,7,8\text{-}(CH_3)_4\ phen\}$

worin:

"phen" Phenanthrolin bedeutet;
"Chel" die in den Ansprüchen 1 und 4 definierte Bedeutung hat;
"bipy" 2,2'-Dipyridyl bedeutet.

**Revendications**

1. Procédé de réduction catalytique de composés nitroaromatiques par transfert d'hydrogène à partir d'oxyde de carbone et d'eau ou à partir de gaz de syhthèse vers un composé nitroaromatique, cette réduction étant catalysée par des complexes de rhodium, iridium, ruthénium et osmium, caractérisé en ce qu'on fait réagir le système $CO + H_2O$ ou le système $(CO + H_2) + H_2O$ avec un composé nitroaromatique de formule:

$$Ar—(NO_2)_x \quad (I)$$

où:

Ar est un groupe aryle ou hétéroaryle, le cas échéant substitué par des groupes inertes;
x est un nombre entier de 1 à 3;

en présence d'un catalyseur de formule:

$$(M\ Chel\ (L)_2)^+X^- \quad (II)$$

ou d'un catalyseur constitué par des composés carbonyles et des chélants de formule:

$$Mz\ (CO)_y + n\ Chel \quad (III)$$

où:

M est égal à Rh, Ir, Ru, Os;
Chel est un composé azoté aromatique bidentate ou tridentate;
L est une molécule d'oxyde de carbone ou d'une oléfine, ou une demi-molécule d'une dioléfine;
$X^-$ est un anion choisi du groupe $Cl^-$, $Br^-$, $I^-$, $PF_6^-$, $BF_4^-$, $B(C_6H_5)_4^-$, $CO_3^{--}$ et $HCO_3^-$;
z est un nombre entier compris entre 4 et 20;
y est un nombre entier compris entre 4 et 30;
n est un nombre entier ou une fraction compris entre 0,1 et 20;

à des températures comprises entre 25°C et 250°C, avec une pression d'oxyde de carbone comprise entre 1 et 150 atmosphères.

2. Procédé selon la revendication 1, caractérisé en ce que le composé nitroaormatique de formule (I) est choisi parmi des dérivés de la catégorie comprenant le benzole, la naphtaline, l'anthracène, le thiényle, le furanne, la pyridine et la quinoline, éventuellement substitués par des groupes inertes, dans les conditions de réaction, par exemple par des groupes alkyle, alkoxyle, amine et analogues.

3. Procédé selon la revendication 1, caractérisé en ce qu'il est mis en oeuvre dans un milieu choisi du groupe comprenant l'eau, l'éthanol, le méthanol, le tétrahydrofuranne et le dioxane, avec addition d'eau.

4. Procédé selon la revendication 1, caractérisé en ce que le composé azoté chélant "Chel" présent dans les catalyseurs de formule (I) et (II) est choisi parmi les substances suivantes: 2,2'-dipyridyle, 4,4'-diméthyl-2,2'-dipyridyle, 1,10-phénanthroline, 5,6-diméthyl-1,10-phénanthroline, 2,9-diméthyl-1,10-phénanthroline, 4,7-diméthyl-1,10-phénanthroline, 3,4,7,8-tétra-méthyl-1,10-phénanthroline, 3,4,5,6,7,8-hexaméthyl-phénanthroline, 2,3,4,7,8,9-hexaméthyl-phénanthroline, 2,4,7,9-tétraméthyl-phénanthroline, phénanthrolines sulfonatées, N,N,N',N'-ortho-phénylène diamine et N,N,N',N'-1,8-diaminonaphtalène et terpyridyle.

5. Procédé selon la revendication 1, caractérisé en ce que l'on utilise pour une mole de composé nitroaromatique (I) $1 \times 10^{-2}$ à $1 \times 10^{-5}$ gramme d'atomes de métal contenu dans le catalyseur.

6. Procédé selon la revendication 1, caractérisé en ce que le composé nitroaromatique (I) est choisi parmi les substances suivantes: nitrobenzol, nitrotoluol, nitroanisol, nitrochlorobenzol, nitroanilin, nitrobenzoamide, nitrobenzonitrile, nitronaphtaline, dinitrobenzol, dinitrotoluol, dinitroaniline, dinitrobenzoamide, nitropyridine, nitrochinoline.

7. Procédé selon la revendication 1, caractérisé en ce que le catalyseur de formule (I) ou (II) est choisi parmi les substances suivantes:

$\{Rh\ 3,4,7,8\text{-}(CH_3)_4\ phen(CO)_2\}B(C_6H_5)_4$
$\{Rh\ 4,7(CH_3)_2\ phen(CO)_2\}B(C_6H_5)_4$
$\{Rh\ phen(CO)_2\}B(C_6H_5)_4$
$Rh_6(CO)_{16}$ + 60 Chel
$Rh_6(CO)_{16}$ + 60 $\{3,4,7,8\text{-}(CH_3)_4\ phen\}$
$Ru_3(CO)_{12}$ + 3 bipy, $Ru_3(CO)_{12}$ + terpyridyl
$Ru_3(CO)_{12}$ + 3 phen, $Ru_3(CO)_{12}$ + 1,5 phen
$Ru_3(CO)_{12}$ + 3 $\{3,4,7,8\text{-}(CH_3)_4\ phen\}$

où

phen signifie phénanthroline;
Chel a la signification définie dans les revendications 1 et 4;
bipy signifie 2,2'-dipyridyle.